Europäisches Patentamt

**European Patent Office**

Office européen des brevets

(19)

(11) Veröffentlichungsnummer : **0 003 358**
**B2**

(12) # NEUE EUROPÄISCHE PATENTSCHRIFT

(45) Veröffentlichungstag der neuen Patentschrift :
03.04.85

(51) Int. Cl.⁴ : **C 07 C 17/04, G 01 N 27/32**

(21) Anmeldenummer : 79100224.9

(22) Anmeldetag : 26.01.79

(54) **Verfahren zur kontinuierlichen Chlorierung von Olefinen in flüssiger Phase sowie Meßanordnung zur Chlorkonzentrationsbestimmung und -regelung als Bestandteile einer Vorrichtung zur Durchführung des Verfahrens.**

(30) Priorität : 26.01.78 DE 2803285

(43) Veröffentlichungstag der Anmeldung :
08.08.79 Patentblatt 79/16

(45) Bekanntmachung des Hinweises auf die Patenterteilung : 18.03.81 Patentblatt 81/11

(45) Bekanntmachung des Hinweises auf die Entscheidung über den Einspruch : 03.04.85 Patentblatt 85/14

(84) Benannte Vertragsstaaten :
**BE DE FR GB NL SE**

(56) Entgegenhaltungen :
**DE-A- 2 540 291**
**DE-A- 2 649 533**
**NL-A- 6 815 959**
**US-A- 3 315 270**
**US-A- 3 933 992**
**Prof. Dr. Ir. P.M.E.M. van der Grinten "Regeltechniek en automatisering in de procesindustrie" Prisma Technica 9, Uitgeverij Het Spectrum N.V. Utrecht/Antwerpen, 1970, Seiten 98-99**
**Gordon M. Barrow, "Physical Chemistry, McGraw-Hill Book Company, New York, etc., 2nd Ed., Seite 742**

(73) Patentinhaber : **WACKER-CHEMIE GMBH**
**Prinzregentenstrasse 22**
**D-8000 München 22 (DE)**

(72) Erfinder : Schmidhammer, Ludwig, Dr. Dipl.-Chem
**Pappelweg 5**
**D-8261 Haiming (DE)**
Erfinder : Selbertinger, Ernst, Ing.Grad.
**Bahnhofstraße 1**
**D-8261 Marktl (DE)**

## Beschreibung

Die Erfindung betrifft ein Verfahren zur kontinuierlichen Chlorierung von Äthylen in flüssiger Phase in Gegenwart eines Chlorierungskatalysators. Dabei wird das Reaktionsgemisch kontinuierlich im Kreislauf durch eine auf 20 bis 60 °C beheizte Reaktionszone sowie Wärmeaustauscher zur Abführung der Reaktionswärme gepumpt. Frisches Äthylen, Chlor und Chlorierungskatalysator wird dem Kreislaufsystem in dem Maße zugeführt, wie katalysatorhaltiges rohes 1.2-Dichloräthan dem Kreislaufsystem entnommen wird.

Derartige Verfahren sind z. B. aus der DE-OS 16 18 273, DE-OS 22 53 720, DE-AS 11 57 592, 15 68 298, 16 68 850, 17 68 367 sowie DE-PS 15 43 108 bekannt. Im allgemeinen wird der entstandene chlorierte Kohlenwasserstoff selbst als Lösungsmittel eingesetzt. Als Chlorierungskatalysatoren werden beispielsweise Eisen III-, Kupfer II-, Wismut III-, Tellur IV- und Zinn IV-chlorid genannt.

Im großtechnischen Betrieb ist es lediglich möglich, das Verhältnis von Äthylen und Chlor vermittels eines Verhältnisreglers relativ grob einzustellen. Chlorüberschuß hat sich einerseits als vorteilhaft erwiesen, um die Bildung von kohlenstoffreichen Harzprodukten und das Durchschlagen von Äthylen ins Abgas zu unterbinden, andererseits wird die Reaktionsausbeute, bezogen auf Chlor, verschlechtert und der Anteil an Chlorsubstitutionsreaktionen verstärkt sich mit zunehmender Chlorkonzentration. Die Aufarbeitung des chlorhaltigen Abwassers aus der Katalysatorwäsche wird erschwert.

Es hat nicht an Versuchen gefehlt, durch Bestimmung der Chlorkonzentration im Reaktionskreislauf rückwirkend Einfluß auf diese zu nehmen. Die Palette reicht von naßchemischer Handanalyse bis zu physikalischen Meßmethoden. Alle bekanntgewordenen Verfahren sind jedoch mit dem Mangel der unbefriedigenden Analysenprobennahme bzw. der zeitverzögernden Aufbereitung der Analkysenprobe behaftet. Auch wenn die ermittelten Meßwerte auf dem schnellsten Wege der Verhältnisregelung Äthylen/Chlor zur Verfügung gestellt wurden, kam es zu unerwünschten Schwankungen der Konzentration an gelöstem Chlor im kontinuierlichen Betrieb. Die erforderlichen vielfältifen Analysenmanipulationen der herkömmlichen Verfahren wirken sich auf die Zuverlässigkeit der ermittelten Meßwerte und damit auf die Reaktionssteuerung negativ aus.

Aufgabe der Erfindung war es somit, ein Verfahren zur Chlorierung zu finden, bei dem vermittels kontinuierlicher und zuverlässiger Erfassung der Chlorkonzentration im Reaktionskreislauf stetig Einfluß auf die Verhältnisregelung von Äthylen und Chlor genommen werden kann. Weiterhin ist es Aufgabe der Erfindung, die Chlorkonzentration im Reaktionskreislauf schwankungsfrei so zu regeln, daß einerseits Chlor im stöchiometrischen Überschuß zu Äthylen, anderseits der Chlorüberschuß nur so gering ist, daß Substitutionsreaktionen unterdrückt werden.

Gegenstand der Erfindung ist ein Verfahren zur kontinuierlichen Chlorierung von Olefinen in flüssiger Phase in Gegenwart eines Chlorierungskatalysators und ständigem Umpumpen des Reaktionsgemisches durch eine erwärmte Reaktionszone, wobei man der flüssigen Phase kontinuierlich etwa in dem Maße Chlor als Führungsgröße und Olefin in etwa stöchiometrisch erforderlichem Verhältnis zuführt, wie Chlorierungsprodukt aus dem Kreislauf entfernt wird, durch Voreinstellung eines Grobverhältnisses und zugeordneter Feindosierung von Olefin, dadurch gekennzeichnet, daß Chlor stets in einem solchen stöchiometrischen Überschuß, bezogen auf Olefin, zugegen ist, daß die Chlorkonzentration in der Kreislaufflüssigkeit 50 bis 500 ppm beträgt, wobei die Chlorkonzentration in der organischen flüssigen Phase von einer Meßanordnung kontinuierlich als proportionales elektrisches Signal erfaßt und einem Regler und einer damit verbundenen Dosiereinrichtung zur Olefinfeindosierung zugeführt wird.

Ein weiterer Gegenstand der Erfindung ist eine Meßanordnung als Bestandteil einer Vorrichtung des Verfahrens zur kontinuierlichen Chlorierung von Äthylen, wobei ein kontinuierliches, der Konzentration von gelöstem Chlor in organischer Phase proportionales elektrisches Signal erzeugt wird, dadurch gekennzeichnet, daß eine Elektrodenkombination bestehend aus einer Platinelektrode als Messelektrode und einer Kalomelelektrode als Bezugselektrode, die, durch ein Diaphragma von dem zu vermezssenden Medium getrennt, als Elektrolytlösung eine wassrige Lösung von Lithiumchlorid enthält in die gelöstes Chlor enthaltende flüssige organische Phase eintaucht.

In einer vorteilhaften Ausführungsform der Meßanordnung tauchen die Elektroden bzw. die Elektrodenkombination in die organische flüssige Phase ein, ohne daß vorher gelöstes bzw. suspendiertes Katalysatormaterial, das üblicherweise ein gegenüber dem Potential $Cl_2/2Cl^-$ niedrigeres Potential aufweist, entfernt wurde. Besonders vorteilhaft ist es, wenn an dem Diaphragma zwischen Meßelektrolytlösung und Chlor enthaltender flüssiger organischer Phase auf die Meßelektrolytlösung ein Flüssigkeitsdruck von $9.8 \cdot 10^{-4}$ bis $4.9 \cdot 10^{-3}$ bar liegt. Dieser Flüssigkeitsdruck kann in besonders vorteilhafter Weise durch eine 1 bis 5 Mol/Liter enthaltende Lithiumchloridlösung erzeugt werden.

Überraschenderweise wird es durch die vorliegende Erfindung ermöglicht, in der organischen flüssigen Phase, in Gegenwart der Katalysatorsuspension und Wasserkonzentrationen in der organischen Phase von im allgemeinen unter 25 ppm, im speziellen Verfahren sogar unter 5 ppm, den Chlorierungsprozeß so zu erfassen und zu regeln, daß keine Schwankungen der Konzentration an gelöstem Chlor während des kontinuierlichen Betriebs der Chlorierungsanlage auftreten. Durch die erfindungsgemäße Meßanordnung als Bestandteil einer Vorrichtung zur Durchführung des Verfahrens wird es möglich, bei Chlorierungsprozessen direkt und kontinuierlich mit großer Zuverlässigkeit die Konzentration an gelöstem Chlor in der flüssigen organischen Phase in Gegenwart des Chlorierungskatalysators zuverlässig zu bestimmen. Durch das erfindungsgemäße Verfahren und die Meßanordnung wird es erstmals ermöglicht, die Chlorierung von Äthylen in Gegenwart von 50 bis 500 ppm Chlorüberschuß mit äußerst geringer Nebenproduktbildung zu führen.

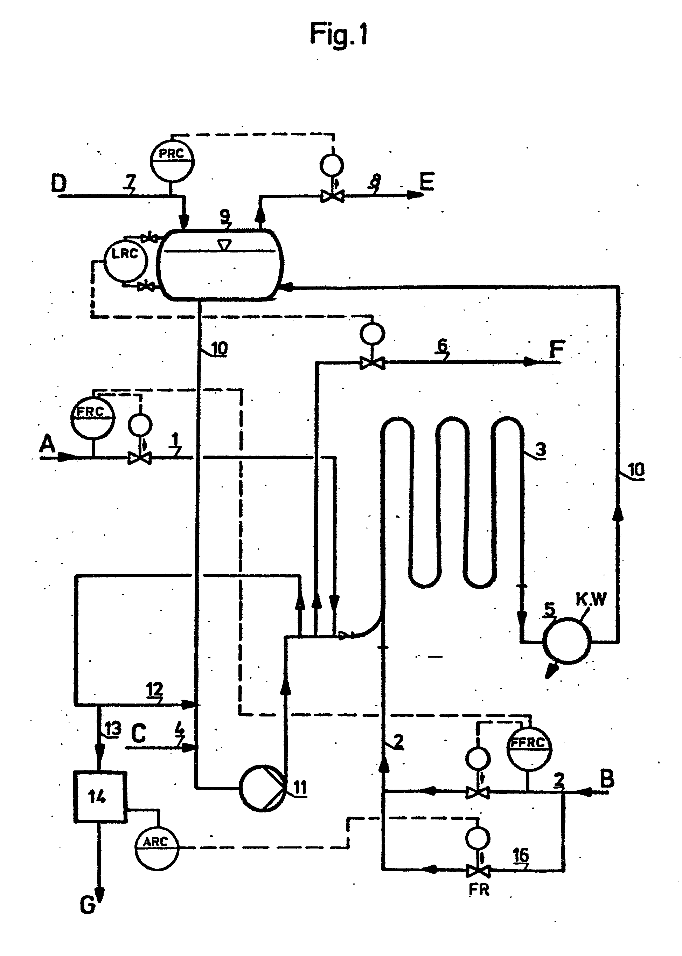

Das erfindungsgemäße Verfahren sowie die Meßanordung sei anhand eines Fließschemas erläutert (Fig. 1).

Über die Leitungen 1,2 und 10 werden dem mit einem Lösungsmittel, wie z. B. einem chlorierten Kohlenwasserstoff, gefüllten Reaktor 3 Chlor als festwertgeregelte Führungsgröße bzw. Äthylen als verhältnisgeregelte Führungsgröße zugeführt. In Gegenwart des Chlorierungskatalysators, der über die Leitung 4 zugeführt wird, als Chlorierungskatalysator kann beispielsweise Eisen III-chlorid eingesetzt werden, wird die Mischung auf die erforderliche Reaktionstemperatur erwärmt. Die Abführung der Reaktionswärme erfolgt im Wärmeaustauscher 5. Über die Leitung 6 wird, in Abhängigkeit vom Füllungszustand im Kreislaufpufferbehälter 9, erzeugtes Chlorierungsprodukt entnommen. Über die Leitung 7 wird der Gasraum des Kreislaufpufferbehälters 9 mit Druckstickstoff gespült, über die Leitung 8 verlassen dieser Spülstickstoff und andere inerte Gase, mit denen Chlor und Äthylen ggf. verunreinigt waren, druckgeregelt das Reaktionssystem in Richtung Abgaswäscher.

Wenn es auch prinzipiell möglich ist, die erfindungsgemäße Meßanordnung im Hauptproduktstrom anzuordnen, so ist es doch vorteilhaft, von der Kreislaufleitung 10 auf der Druckseite der Kreislaufpumpe 11 eine Bypassleitung 12 anzuordnen, die auf der Saugseite der Kreislaufpumpe 11 in die Hauptproduktstromleitung 10 zurückgeführt wird. Die Elektrodenmeßanordnung kann in der Bypassleitung angeordnet werden, vorzugsweise wird jedoch wiederum von der Bypassleitung eine weitere Leitung 13 abgezweigt, die aufgrund ihres geringen Querschnittes im Verhältnis zur Bypassleitung lediglich ca. 50 1/Stunde aus der Bypassleitung abzweigt und der Chlorkonzentrationsmeßanordnung 14 zuführt. Der Abfluß aus der Meßanordung wird direkt der Katalysatorwäsche zugeführt.

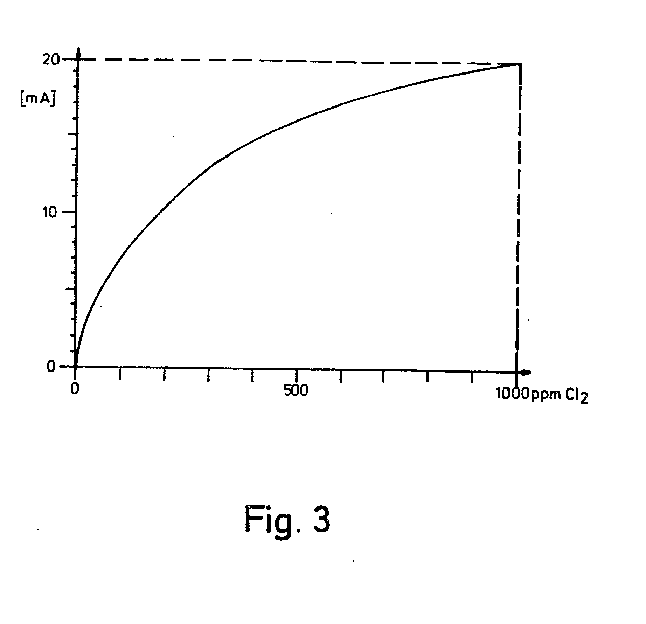

Das in der Chlorkonzentrationsmeßanordnung erzeugte elektrische Signal, ein der Chlorkonzentration proportionales elektrisches Potential, wird in einen, dem chemischen Potential entsprechenden Strom umgesetzt (im allgemeinen zwischen 0 und 20 mA) (vgl. Fig. 3) und damit über einen Regler und ein zugeordnetes Feinregulierventil 16 proportionale Mengen Äthylen in die Äthylenhauptzuhrungsleitung 2 zusätzlich zugeführt. Damit können Schwankungen im Verhältnis Chlor zu Äthylen, wie sie bei der Grobverhältnisregelung auftreten können, durch die Feinregulierung ausgeglichen werden und der vorgegebene Sollwert an freiem, überschüssigem Chlor in der Kreislaufflüssigkeit konstantgehalten werden.

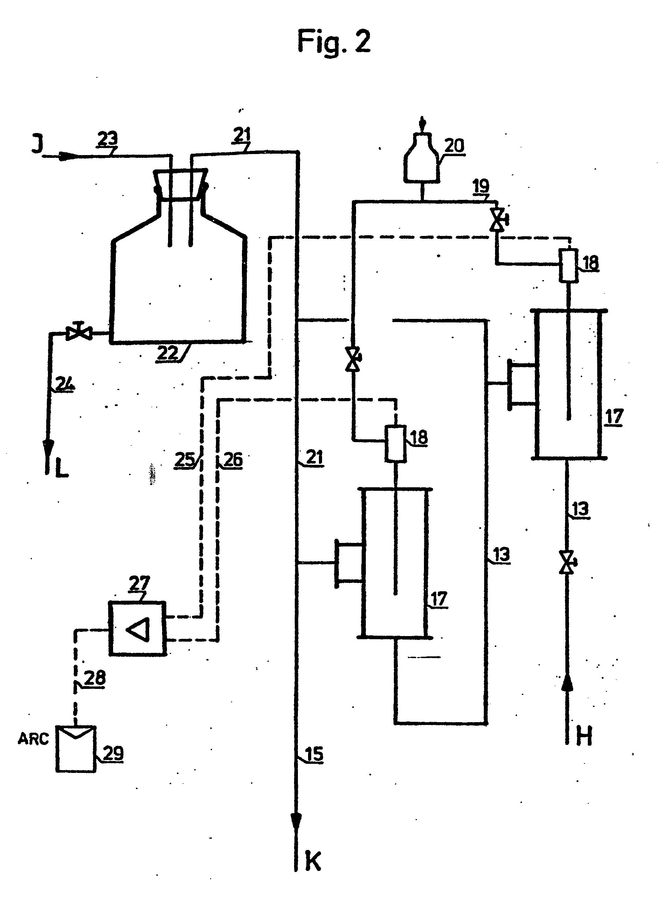

In Fig. 2 ist ein mögliches Anordnungsschema für das Chlorkonzentrationsmeßgerät gegeben. Um bei etwaigen Störungen bei der Potentialmessung keine Schwankungen im Chlorüberschuß der Hauptstromleitung hinnehmen zu müssen, ist der Durchlaufmeßgeber 17 samt Elektrodenkombination zweifach mit entsprechender Umschaltung installiert, obwohl die Zuverlässigkeit der erfindungsgemäßen Meßanordnung auch ohne weiteres eine einfache Ausführung zulassen würde. Über die Leitung 13 treten ca. 50 1/Stunde Kreislaufflüssigkeit in den Durchlaufmeßgeber, der beispielsweise aus Geräteglas gefertigt sein kann. Im Durchlaufmeßgeber befindet sich die Elektrodenkombination.

Als Elektroden wird eine Kombination aus Meß- und Bezugselektrode eingesetzt. Als Bezugselektrode eignet sich beispielsweise eine Platinelektrode, als Meßelektrode eine Kalomelelektrode. Die Elektroden können auch in einer Einstabmeßkette zusammengefaßt werden. Solche Elektroden bzw. Kombinationen sind handelsüblich. Vorteilhaft hat es sich erwiesen, als inneren Elektrolyt der Meßelektroden eine Lithiumchloridlösung mit Konzentrationen zwischen 1 und 5 Mol Lithium/Liter Wasser einzusetzen. Durch diese Maßnahme kann das Verkrusten der Glasmembranen der Meßelektroden bei Anwesenheit von Schwebestoffen, wie beispielsweise Katalysatoren, weitgehend vermieden werden.

Über die Leitung 13 gelangt die Kreislaufflüssigkeit in den zweiten, für Reservezwecke vorgesehenen, Durchlaufmeßgeber und von dort in den freien Ablauf zur Katalysatorwäsche. Zur Aufrechterhaltung eines konstanten Drucks im Durchlaufmeßgeber wird dort über Leitung 21 ein Druckausgleich mit einem Gaspuffer angelegt, der mit einem konstanten Stickstoffgasstrom mit beispielsweise $3 \cdot 10^{-2}$ bar versorgt wird.

Das gemessene Chlor-Redoxpotential wird über die abgeschirmten Meßleitungen 25 bzw. 26 auf ein Meßgerät übertragen, beispielsweise ein Betriebs-pH-Meter. Über Leitung 28 wird das Meßsignal auf den Regler ARC übertragen, der über das zugeordnete Feinregulierventil die zu chlorierende Komponente in die Leitung 2 (Fig. 1) feinreguliert. Das in der Meßanordnung festgestellte elektrische Potential wird in einem Potentialverstärker in einen proportionalen Strom umgesetzt, der wiederum proportional der Chlorkonzentration in der Kreislaufflüssigkeit ist. Eine typische Eichkurve, die den Stromausgang eines Potentialverstärkers mit der Chlorkonzentration korrigiert, ist in Fig. 3 dargestellt. Im allgemeinen können Chlorkonzentrationen zwischen 0 und 2.000 ppm auftreten, vorteilhafterweise wird die Chlorkonzentration auf einen Wert zwischen 50 und 500 ppm eingestellt, insbesondere arbeitet man bei Chlorkonzentrationen an freiem Chlor zwischen 150 und 300 Gew.ppm, eine Feinregulierung der Konzentration an freiem Chlor zwischen 200 und 250 ppm ist erfindungsgemäß ohne weiteres möglich und wird zur Vermeidung der Bildung von Chlorsubstitutionsreaktionen in besonderen Fällen angewandt.

Zur Erläuterung des Verfahrens dient das folgende Beispiel:

700 m³/h 1.2-Dichloräthan mit 250 ppm Eisen III-chlorid als Chlorierungskatalysator werden als Lösungsmittel mit Hilfe der Pumpe 11 im Kreislauf durch den Wärmetauscher 5, Pufferbehälter 9, Reaktor 3 mit insgesamt 16 m³ Inhalt und die Kreislaufleitung 10 geführt. 3.000 Nm³/h Flüssigchlor werden über Leitung 1 zugeführt. Über die Verhältnisregelung FFRC werden in Abhängigkeit von der Chlordurchflußmenge FRC 2.900 Nm³/h Äthylen über Leitung 2 in das Reaktionssystem eingebracht. Die Temperatur im Reaktor 3 wird auf 40 bis 50 °C gehalten. Im Wärmetauscher 5 wird die entstehende Reaktionswärme abgeführt.

Die Analysenringleitung 12 wird von ca. 1.000 1/Stunde Kreislaufflüssigkeit durchströmt, wovon 50 1/Stunde vom Analysator 14 fortlaufend auf den Gehalt an freiem Chlor untersucht werden. Der Sollwert an überschüssigem, freiem Chlor wird bei ARC auf 220 Gew.ppm eingestellt. Gehalten wird dieser Sollwert durch automatische Zugabe von im Schnitt ca. Nm³/h Äthylen über das Feinregulierventil FR in Leitung 16. Das entstehende 1,2-Dichloräthan wird in Abhängigkeit vom geregelten Niveau LRC im Kreislaufpufferbehälter 5 über Leitung 6 abgezogen und der Katalysatorwäsche zugeführt. Es enthält 250 ppm FeCl₃, 300 ppm HCl, 220 ppm Chlor und < 10 ppm Äthylen.

Die Abgase verlassen das Reaktionssystem druckgeregelt über Leitung 8 zusammen mit dem über Leitung 7 zugeführten Stickstoffspülstrom in Richtung Abgaswäscher. Dieser Abgasstrom enthält neben Stickstoff ca. 0,03 Vol.% Äthan (im Äthylen enthalten) und < 100 Vol.ppm Äthylen. Der Äthylenumsatz, bezogen auf eingesetztes Äthylen, beträgt somit 99,99 %, die Äthylenausbeute liegt bei 99,7 %. Der Anfall an Nebenprodukten, wie Äthylchlorid und 1.1.2-Trichloräthan sowie Polymerisat, erreicht insgesamt 0,4 Gew.%, bezogen auf erzeugtes Roh-Dichloräthan.

Selbstverständlich läßt sich die erfindungsgemäße Chlornachweismethode ohne weiteres auch auf andere Chlorierungsreaktionen anwenden, bei denen ein exakt einzuhaltender Chlorüberschuß zur Prozeßoptimierung notwendig ist bzw. bei denen man nach erfolgter Chlorierung den Chlorüberschuß durch eine in Abhängigkeit vom Chlormeßwert gesteuerte Zugabe von Chlorakzeptoren vor der weiteren Aufarbeitung des Chlorierungsproduktes zur Verhinderung von Nebenreaktionen in möglichst wirtschaftlicher Weise wieder entfernen muß.

Gemäß DE-AS 1 157 592 arbeitet man bei der Herstellung von 1.2-Dichloräthan mit einem Chlorüberschuß von 0,1 bis 10 %, vorzugsweise 2 bis 5 %, im Hauptreaktor, muß aber zur Reduzierung der substituierenden Weiterchlorierung von Dichloräthan das überschüssige Chlor in einem unter Druck stehenden Nachreaktor mit Äthylen im Überschuß wegreagieren, wobei der dazu verwendete Äthylenstrom nach Durchleiten durch den Nachreaktor im Hauptreaktor mit Chlorüberschuß zur Reaktion gebracht wird.

Das folgende Vergleichsbeispiel verdeutlicht das Maß der Nebenproduktbildung durch Substitutionsreaktionen bei einem Chlorüberschuß von 1 200 ppm:

700 m³/h 1.2-Dichloräthan mit 250 ppm Eisen III-chlorid als Chlorierungskatalysator werden als Lösungsmittel mit Hilfe der Pumpe 2 im Kreislauf durch den Reaktor 3, den Wärmetauscher 5 und den Pufferbehälter 9 mit insgesamt 16 m³ Inhalt und die Kreislaufleitung 10 geführt. 3.000 Nm³/h Flüssigchlor werden über Leitung 1 zugeführt. Über die Verhältnisregelung FFRC werden in Abhängigkeit von der Chlordurchflußmenge 2.720 Nm³/h Äthylen über Leitung 2 in das Reaktionssystem eingebracht. Die Temperatur im Reaktor 3 wird auf 40 bis 50 °C gehalten. Im Wärmetauscher 5 wird die entstehende Reaktionswärme abgeführt.

Das entstehende 1.2-Dichloräthan wird in Abhängigkeit vom geregelten Niveau LRC im Kreislaufpufferbehälter 9 über Leitung 6 abgezogen und der Katalysatorwäsche zugeführt. Es enthält 250 ppm FeCl₃, 1.800 ppm HCl, 1.200 ppm Chlor und < 10 ppm Äthylen.

Die Abgase verlassen das Reaktionssystem druckgeregelt (PRC) über Leitung 8 zusammen mit dem über Leitung 7 zugeführten Stickstoffspülstrom von ca. 10 m³/h in Richtung Abgaswäscher. Dieser Abgasstrom enthält neben Stickstoff ca. 0.03 Vol.% Äthan (im Athylen enthalten) und < 100 Vol.ppmm Äthylen.

Der Äthylenumsatz bezogen auf eingesetztes Äthylen, beträgt somit 99,99 %, die Äthylenausbeute liegt bei 99.7 %. Der Anfall an Nebenprodukten, wie Äthylchlorid, 1.1.2-Trichloräthan, Tetrachloräthan sowie Polymerisat erreicht insgesamt 1,0 Gew.%, bezogen auf erzeugtes Roh-Dichloräthan.

**Ansprüche**

1. Verfahren zur kontinuierlichen Chlorierung von Olefinen in flüssiger Phase in Gegenwart eines Chlorierungskatalysators und ständigem Umpumpen des Reaktionsgemisches durch eine erwärmte Reaktionszone, wobei man der flüssigen Phase kontinuierlich etwa in dem Maße Chlor als Führungsgröße und Olefin in etwa stöchiometrisch erforderlichem Verhältnis zuführt, wie Chlorierungsprodukt aus dem Kreislauf entfernt wird, durch Voreinstellung eines Grobverhältnisses und zugeordneter Feindosierung von Olefin, dadurch gekennzeichnet, daß Chlor stets in einem solchen stöchiometrischen Überschuß, bezogen auf Olefin, zugegen ist, daß die Chlorkonzentration in der Kreislaufflüssigkeit 50 bis 500 ppm beträgt, wobei die Chlorkonzentration in der organischen flüssigen Phase von einer Meßanordnung kontinuierlich als proportionales elektrisches Signal erfaßt und einem Regler und einer damit verbundenen Dosiereinrichtung zur Olefinfeindosierung zugeführt wird.

2. Verfahren nach Anspruch 1, dadurch gekennzeichnet, daß eine Meßanordnung als Bestandteil der Vorrichtung zur Durchführung des Verfahrens verwendet wird, die ein elektrisches Signal erzeugt, das der Konzentration von gelöstem Chlor in organischer Phase proportional ist, wobei eine Elektrodenkombination, bestehend aus

a) einer Platinelektrode als Meßelektrode und

b) einer Kalomelelektrode als Bezugselektrode die, durch ein Diaphragma von dem zu vermessenden Medium getrennt, als Elektrolytlösung eine wäßrige Lösung von Lithiumchlorid enthält, in die gelöstes Chlor enthaltende flüssige organische Phase eintaucht.

## Claims

1. Process for the continuous chlorination of olefins in the liquid phase in the presence of a chlorination catalyst and with constant circulation of the reaction mixture through a heated reaction zone, in which process chlorine, as the reference variable, and olefin are admitted to the liquid phase continuously, in approximately the stoichiometrically required ratio, in proportion as the chlorination product is removed from the circulatory system, as a result of pre-setting a rough ratio and of co-ordinated precision admittance in doses of the olefin, characterized in that chlorine is present always in such a stoichiometric excess, based on the olefin, that the concentration of chlorine in the circulating liquid amounts to from 50 to 500 ppm, with the concentration of chlorine in the organic liquid phase being continuously recorded by a measuring arrangement as a proportional electric signal which is passed to a regulator connected to a dosing device for the precise admittance in doses of the olefin.

2. Process according to claim 1, characterized in that a measuring arrangement is used as a component part of an apparatus which is used for performing the process, said measuring arrangement generating a continuous electrical signal proportional to the concentration of dissolved chlorine in the organic phase, wherein a combination of electrodes comprising

a) a platinum electrode as a measuring electrode and

b) a calomel electrode as a reference electrode which is separated from the medium to be measured by a diaphragm and contains an aqueous lithium chloride solution as an electrolyte solution, is immersed in the liquid organic phase containing dissolved chloride.

## Revendications

1. Procédé pour chlorer en continu des oléfines en phase liquide, en présence d'un catalyseur de chloration et avec circulation continue du mélange réactionnel par pompage à travers une zone réactionnelle chauffée, du chlore, grandeur directrice, et de l'oléfine étant amenés continuellement à la phase liquide, approximativement dans le rapport stœchiométrique, à peu près dans la mesure où l'on retire du circuit le produit de chloration, par pré-réglage d'un rapport grossier et apport précis adjoint d'oléfine, procédé caractérisé en ce que le chlore est constamment présent en un excès stœchiométrique, par rapport à l'oléfine, tel que la concentration en chlore du liquide en circulation soit de 50 à 500 ppm, la concentration en chlore de la phase liquide organique étant continuellement saisie par un dispositif de mesure sous la forme d'un signal électrique proportionnel et transmise à un régulateur lié à un dispositif d'alimentation qui introduit l'oléfine à un débit précis.

2. Procédé selon la revendication 1 caractérisé en ce qu'on utilise, en tant qu'élément de l'appareil pour l'exécution du procédé, un dispositif de mesure créant un signal électrique proportionnel à la concentration du chlore dissous dans la phase organique, dispositif caractérisé en ce qu'une association d'électrodes comprenant :

a) comme électrode de mesure une électrode de platine et

b) comme électrode de référence une électrode au calomel qui contient, comme solution électrolytique, une solution aqueuse de chlorure de lithium séparée par un diaphragme du milieu sur lequel doit être effectuée la mesure, plonge dans la phase organique liquide contenant le chlore dissous.

# Fig.1

# Fig. 2

Fig. 3